# EUROPEAN PATENT APPLICATION

(11) **EP 4 523 668 A1**
(43) Date of publication of application: **19.03.2025**
(21) Application number: 23197434.6
(22) Date of filing: 14.09.2023
(51) Int. Cl.: A61F 13/534

(54) **ADAPTIVE HYGIENE ABSORBENT PRODUCT**

(71) Applicant: Corman SpA, 20084 Lacchiarella (IT)
(72) Inventor: MANTOVANI, Giorgio, 20084 Lacchiarella (IT); MASSAGLI, Matteo, 20084 Lacchiarella (IT)
(74) Representative: Wächtershäuser & Hartz Patentanwaltspartnerschaft mbB

(57) **Abstract**

The invention provides an absorbent product comprising a fluid-permeable top sheet, a fluid-impermeable back sheet, and an absorbent core positioned between said top sheet and said back sheet in a central portion of the absorbent product,
said absorbent core comprising an absorbent layer and a cover sheet covering the absorbent layer, said cover sheet extending beyond the absorbent layer on at least two opposing sides of the absorbent layer, characterized in that said cover sheet is an extensible fibrous sheet.

## Description

### FIELD OF THE INVENTION

The invention relates to an absorbent product, such as a sanitary napkin, panty liner, or incontinence pad, comprising a fluid-permeable top sheet, a fluid-impermeable back sheet, and an absorbent core positioned between said top sheet and said back sheet.

### BACKGROUND OF THE INVENTION

Hygiene absorbent products comprising multiple layers are known. A pad with an absorbent core consisting of two layers whereby the lower layer is narrower than the upper one is described in EP0590675 A1 (Van Gompel, Kimberly-Clark, 1992). This document also describes elasticized side flaps of the pad. This design fails to provide an irreversibly absorbing lower pad having a high concentration of superabsorbent polymer covered by a fibrous layer comprising longer fibers that those on said lower layer and able to extend in cross direction without losing its integrity. Further, the ability to form a containment for discharged body fluid is insufficient. Diapers having side flaps that are capable of providing containment for discharged body fluids are described in US 3,860,003 (Buell, Procter and Gamble, 1973).

The need to control the pH value in absorbent hygiene products is known. Typically, the superabsorbent polymer contained in absorbent cores can control the pH value in certain limits for avoiding the effects of bacterial decomposition of urine leading to ammonia release. In some products, the addition of so called crosslinked and curled cellulosic fibers using citric acid technology can support the pH balance. However, such solutions have the disadvantage that the time needed to develop the pH buffering action is quite long, and the ammonia neutralization and pH buffering capacity limited. Therefore, there remains the problem of avoiding decomposing urine and development of ammonia odor.

Departing from the prior art, it is a technical problem of the invention to provide an absorbent product that provides good containment even after the absorbent core has swollen due to uptake of body fluid, such as urine or blood. It is a further problem to provide an absorbent product that maintains sufficient stability and integrity even after the absorbent core has swollen due to uptake of body fluid, such as urine or blood. Moreover, it is a technical problem of the invention to improve the control of urine decomposition and associated odor formation.

### SUMMARY OF THE INVENTION

In order to solve these problems, the invention provides:
1) An absorbent product comprising a fluid-permeable top sheet, a fluid-impermeable back sheet, and an absorbent core positioned between said top sheet and said back sheet in a central portion of the absorbent product,
   said absorbent core comprising an absorbent layer and a cover sheet covering the absorbent layer, said cover sheet extending beyond the absorbent layer on at least two opposing sides of the absorbent layer, said cover sheet being preferably an extensible fibrous sheet.
2) The absorbent product according to (1), characterized in that said cover sheet is an extensible fibrous sheet; and/or the fibers of the cover sheet have a mean length by number L(n) of at least 5 mm, preferably at least 8 mm, more preferably at least 10 mm, even more preferably at least 12 mm, and most preferably at least 15 mm.
3) The absorbent product according to (1) or (2), wherein the absorbent product has an elongated and essentially flat shape or is capable of accommodating an elongated flat shape, wherein the cover sheet extends beyond the absorbent layer at least along the lateral sides of the absorbent layer, the lateral sides extending essentially parallel to the elongation direction.
4) The absorbent product according to any one of (1) to (3), having a longitudinal x-direction or axis extending along the elongation (long) direction of the elongated absorbent product, a cross (or transverse) y-direction or axis within the flat plane orthogonal to longitudinal x-direction, and a thickness z-direction or axis orthogonal to both the longitudinal and transverse directions,
   said cover sheet having a width in cross direction at least 4 mm, preferably at least 12 mm, larger than the width of the absorbent layer as viewed along the z-direction on the product in planar shape, at least at a central part, along the x-direction, of the product, preferably along the entire length in x-direction of the absorbent layer.
5) The absorbent product according to any one of (1) to (4), wherein the cover sheet is affixed to the back sheet at its portions extending beyond the absorbent layer so as to enclose the absorbent layer between the cover sheet and the back sheet.
6) The absorbent product according to any one of (1) to (5), wherein said cover sheet, before attaching or integrating it into said absorbent product, is extensible, at least in cross direction, by at least 20%, preferably at least 30%, more preferably at least 40% in length without breaking.
7) The absorbent product according to any one of (1) to (6), wherein at least 70 % by weight of the fibers of the cover sheet have a length of at least 8 mm, preferably at least 10 mm.
8) The absorbent product according to any one of (1) to (7), wherein the cover sheet is a sheet of fluffy wadding of said fibers, and/or said fibers are interconnected exclusively by mechanical forces selected from fiber-to-fiber friction, entangling, and interlacing, wherein under tension applied to the cover sheet, the fibers can move relatively to each other without breakage of said cover sheet.
9) The absorbent product according to any one of (1) to (8), wherein said fibers are fibers of polyethylene (PE), polypropylene (PP), polyethylene terephthalate (PET), cellulosic, or mixtures thereof, preferably said fibers are cellulosic fibers.
10) The absorbent product according to (9), wherein said cellulosic fibers are cotton fibers, pulp fibers, and/or viscose fibers, preferably cotton fibers.
11) The absorbent product according to any one of (1) to (10), wherein said cover sheet contains 20 % by weight or less of superabsorbent polymer, preferably from 1 to 15 % by weight of superabsorbent polymer.
12) The absorbent product according to any one of (1) to (11), wherein said cover sheet has a basis weight of 20 to 200 g/m², preferably from 30 to 120 g/m².
13) The absorbent product according to any one of (1) to (12), wherein said absorbent layer comprising particles of superabsorbent polymer and at most 10 % by weight of fibers shorter than 10 mm, preferably shorter 8 mm.
14) The absorbent product according to any one of (1) to (13), wherein said absorbent layer comprises at least 40 % by weight, preferably at least 50 % by weight of particles of a superabsorbent polymer.
15) The absorbent product according to any one of (1) to (14), wherein the absorbent layer, when saturated with aqueous 0.9% NaCl solution, expands in z-direction (thickness) at least by 3 times its (original) thickness.
16) The absorbent product according to any one of (1) to (15), wherein said absorbent layer is bonded to said back sheet.
17) The absorbent product according to any one of (1) to (16), wherein said top sheet and said back sheet are bonded to each other along the periphery of the absorbent product to form a seal area 13.
18) The absorbent product according to a (17), wherein said seal area 13 comprises lateral portions extending on and along lateral sides of said absorbent product, said lateral portions containing elastic elements arranged in x-direction and in extended condition when the absorbent product accommodates a flat planar shape, said elastic elements exerting a contracting force on the lateral portions to contract them and to form the absorbent product to a cup-shape when said elastic elements are relaxed.
19) The absorbent product according to (18), wherein, when in a flat shape/state of the absorbent product, the elastic elements are extended to at least 120%, preferably at least 150%, in length compared to a relaxed state of the elastic elements.
20) The absorbent product according to (18) or (19), wherein the elastic element closest to the absorbent layer in y-direction of the product in planar shape is distanced from the absorbent layer in cross direction by at least 5 mm, preferably by at least 7 mm, when the absorbent product is formed into a flat shape.
21) The absorbent product according to any one of (1) to (20), wherein said absorbent product contains at least 0.1 g of a pH regulating agent, preferably applied to the bottom side of the top sheet; said pH regulating agent may be a solid organic acid or an acidic salt of an organic acid, such as citric acid, tartaric acid, ascorbic acid, sodium dihydrogen citrate, and/or mono-sodium tartrate, preferably sodium dihydrogen citrate.
22) The absorbent product according to any one of (1) to (21), wherein said absorbent product contains an odor trapping material, such as a cyclodextrin or activated carbon.
23) An absorbent product comprising a fluid-permeable top sheet, a fluid-impermeable back sheet, and an absorbent core positioned between said top sheet and said back sheet in a central portion of the absorbent product,
   said absorbent core comprising an absorbent layer and a cover sheet covering the absorbent layer, said cover sheet extending beyond the absorbent layer on at least two opposing sides of the absorbent layer,
   wherein said cover sheet is an extensible fibrous sheet extensible by at least 20 %, at least in cross direction, without breaking, said cover sheet comprising, preferably consisting of, cellulosic fibers (preferably cotton fibers) and optionally superabsorbent polymer particles.
24) An absorbent product comprising a fluid-permeable top sheet, a fluid-impermeable back sheet, and an absorbent core positioned between said top sheet and said back sheet in a central portion of the absorbent product,
   said absorbent core comprising an absorbent layer and a cover sheet covering the absorbent layer, said cover sheet extending beyond the absorbent layer on at least two opposing sides of the absorbent layer,
   wherein said cover sheet is an extensible fibrous sheet extensible by at least 20 %, at least in cross direction, without breaking, said cover sheet comprising, preferably consisting of, cotton fibers and optionally superabsorbent polymer particles, and
   wherein fibers of the cover sheet are interconnected exclusively by mechanical forces to allow movement of interconnected fibers relative to each other.
25) An absorbent product comprising a fluid-permeable top sheet, a fluid-impermeable back sheet, and an absorbent core positioned between said top sheet and said back sheet in a central portion of the absorbent product,
   said absorbent core comprising an absorbent layer and a cover sheet covering the absorbent layer, said cover sheet extending beyond the absorbent layer on at least two opposing sides of the absorbent layer,
   wherein said cover sheet is an extensible fibrous sheet extensible by at least 20 %, at least in cross direction, without breaking, said cover sheet comprising, preferably consisting of, cellulosic fibers (preferably cotton fibers) and optionally superabsorbent polymer particles, and
   wherein fibers of the cover sheet are interconnected exclusively by mechanical forces to allow movement of interconnected fibers relative to each other,
   wherein said top sheet and said back sheet are bonded to each other along the periphery of the absorbent product to form a seal area 13, said seal area 13 comprises lateral portions extending on and along lateral sides of said absorbent product, said lateral portions containing elastic elements arranged in x-direction and in extended condition when the absorbent product accommodates a flat planar shape, said elastic elements exerting a contracting force on the lateral portions to contract them and to form the absorbent product to a cup-shape when said elastic elements are relaxed.
26) The absorbent product according to (25), wherein the elastic element closest to the absorbent layer in y-direction of the product in planar shape is distanced from the absorbent layer in cross direction by at least 5 mm, preferably by at least 7 mm, when the absorbent product is formed into a flat shape.
27) The absorbent product according to any one of (23) to (26), wherein the fibers of the cover have a mean length by number L(n) of at least 5 mm, preferably at least 8 mm, more preferably at least 10 mm, even more preferably at least 12 mm, and most preferably at least 15 mm; or the fibers of the cover sheet comprise at least 50 % by weight fibers of a length of at least 7 mm, preferably at least 10 mm, more preferably at least 12 mm, even more preferably at least 14 mm, and most preferably at least 17 mm.

The absorbent product of the invention has a cover sheet positioned below the top sheet and covering the absorbent layer, wherein the cover sheet has a special structure that allows extending it without losing integrity. In use of the absorbent product, the absorbent layer swells by uptake of body fluid, whereby the thickness of the absorbent layer may increase several-fold. The swelling provides stress and/or deformation on the cover sheet. The structure of the cover sheet of the invention, that is extensible e.g. by comprising a large proportion of long fibers (as further described below), allows movement of the fibers relative to each other without losing contact, whereby the fibrous sheet is extensible and maintains integrity.

Moreover, the absorbent product of the invention can, in spite of the swelling of the absorbent layer, maintain a cup-shape when in contact with the body and thus provides good containment to prevent leakage of body fluid. Furthermore, the absorbent product can, in a preferred embodiment, better control odor formation due to decomposition of urine absorbed by the product.

### DRAWINGS

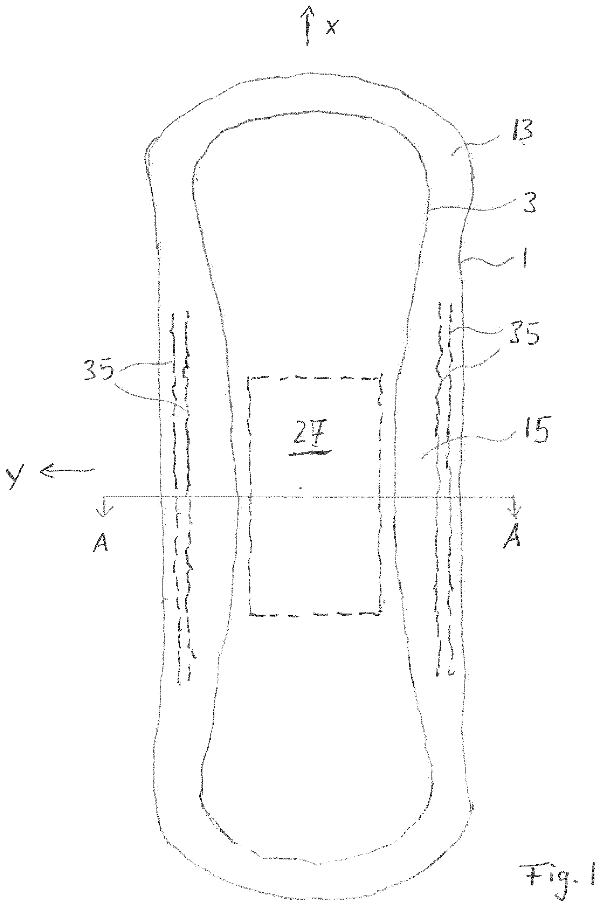

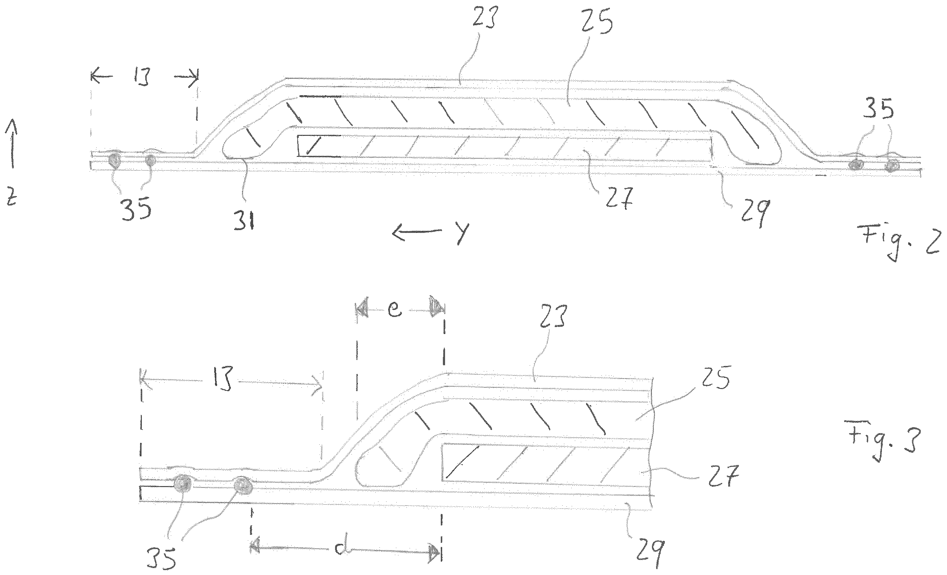

Fig. 1 Absorbent product as deformed to be planar. View on the inside, i.e. on the top sheet, wherein the back sheet hidden. Letter x indicates the longitudinal direction, y indicates the cross direction. The (not indicated) z-direction is orthogonal to the former directions. The dashed lines indicate the location of the absorbent layer 27 below the top sheet and cover sheet that is not shown. Numeral 15 indicates lateral sides containing extended rubber elements 35 that pull the side flaps together in x-direction to move the side flaps upwards (towards the viewer) in z-direction to form a cup-shape of the absorbent product when the deformation force is released. Line A-A indicates the position of the cross-section of the absorbent product shown in Fig. 2.
Fig. 2 Cross-section of absorbent product of the invention along line A-A of Fig. 1. Numerals 23, 25, 27 and 29 indicate the top sheet, the cover sheet, the absorbent layer, and the back sheet, respectively. 13 indicates the seal area where the cover sheet bonds to the back sheet.
Fig. 3 shows an enlarged portion of the cross-section of Fig. 2 defining distances d and e and highlighting seal area 13 containing elastic elements 35.

### DETAILED DESCRIPTION OF THE INVENTION

The absorbent product of the invention comprises at least the following main elements, in this order, from the inner side (facing the body of the user in use) to the outer side: (i) a top sheet, (ii) an absorbent core, and (iii) a back sheet. The absorbent core is positioned between said top sheet and said back sheet at least in a central portion of the absorbent product. Thus, the absorbent core does not have the same extensions as the top and the back sheet, but is smaller. The absorbent core, in turn, comprises at least the following two elements: an absorbent layer closer to the back sheet and the cover sheet that covers the absorbent layer and faces the top sheet.

The top sheet has the main function of allowing permeation of fluid released from the body of a user through it. The absorbent core has the main function of absorbing and retaining the body fluid. The back sheet has the main function of preventing release (leakage) of fluid from the product on the side facing the outside (i.e., the side opposite to that facing the body of the user). Generally, the top sheet and the back sheet further function as outer physical boundary of the product, an envelope that encloses the absorbent core. Each of elements (i) to (iii) may comprise or consists of more than one layer(s). For example, the top sheet may be two-layered sheet. The absorbent product may further comprise an acquisition and distribution layer (ADL) between the top sheet and the cover sheet, for distributing fluid transmitted through the top sheet.

The product generally has an elongated flat shape or can be easily deformed to an elongated flat planar shape, with a longitudinal or x-dimension that extends along the elongation direction of the product within the plane of the flat shape. The y-direction (also referred to as cross, width or transverse direction) is also within the plane of the flat shape, but is orthogonal to the longitudinal x-direction. The y-direction is along the short dimension of the product. The x- and y-axes are indicated in Fig. 1 . The z-dimension is the thickness direction that is orthogonal to both the x- and y-dimension, shown in Figs. 2 and 3. The product is flexible to be able to adjust to the body shape of the user.

Generally, at least layers (i) and (iii) are or comprise a sheet. Also, the absorbent core comprises a sheet, namely the cover sheet and the absorbent layer. The components of the absorbent product and materials from which they can be made are described next.

### Top sheet

The top sheet should be soft and gentle as it contacts the skin of the user. Moreover, it should allow fast acquisition of fluid from the body of the user and quickly passing the fluid on to the inner layers of the absorbent product. The top sheet should remain or feel dry after passing the fluid to the inner layers. The top sheet of the product of the invention is fluid permeable. In the invention, the fluid is a liquid, preferably a body exudate such as blood or urine. Fluid permeability of the top sheet may be determined using water or, better, aqueous NaCl solution.

The top sheet of the absorbent product may be a nonwoven, e.g. made of fibers of a polypropylene (PP) or PP/PE fibers, carded or spunbonded and/or air-trough bonded. Such top sheet nonwovens are inter alia described in EP 91905693 B1, US3489148 A, US8530722, or EP2399558. Alternatively, the top sheet may be made by air-through bonding technology which provides a significant improvement towards even softer, bulkier, and skin-friendly nonwovens. Examples of such materials are described in EP1369519, in EP 2 708 623 and WO2014022988. A method for producing such high loft nonwovens is described in EP3246444.

Preferably, the top sheet is made of a material that is bio-based and biodegradable. The top sheet preferably is or comprises a sheet that is a fabric made of fibers of cellulose, e.g. cotton or viscose fibers, or of fibers of a cellulose derivative, e.g. fibers made of cellulose esters such as described in DE3312022 A1 and DE3246417 A1. Preferably, the fabric is a nonwoven fabric made of cellulose fibers. The nonwoven may be produced by hydroentanglement from fibers (also referred to in the art as "spunlacing"). The fibers (such as viscose fibers) used for hydroentanglement may have a median length of 5 to 30 mm, preferably from 10 to 20 mm (by number). Fiber length can be measured as described below. The median length of the fibers may be determined from the lengths of fibers of a representative sample of fibers. Suitable top sheets are offered in the market for example by Fama Jersey S,p,A. company e.g., as Maspun 35100 C P1.

The top sheet may have perforations allowing body exudate to be quickly transferred to lower layers, notably the absorbent core. The perforations may have a size of from 0.2 to 10 mm², preferably of from 0.5 to 5 mm². The perforations may be preferably located in an area of the absorbent product, that contacts body parts from which body exudate is to be expected.

The top sheet may be at least partially coated with, and may thus contain a layer of, a hydrophilic polymer, preferably at least on its outer surface facing the body of the user. Examples of hydrophilic polymers are carboxymethyl cellulose, alginates or other hydrogels like gelatin, chitosan, or hyaluronic acid or a salt of the afore-mentioned polymers.

The basis weight of the top sheet nonwoven may be from 20 to 50, preferably 30 to 40 g/m². Its average density may be from 80 to 160, preferably from 100 to 140 kg/m³. The wicking rate of this top sheet material may be 0 measured by EDANA ERT 10.3-99. The thickness of the top sheet is generally within a range of from 0.1 to 4 mm, preferably from 0.2 to 2 mm.

### Back sheet

The back sheet together with top sheet constitute an envelope of the absorbent product of the invention. The role of the back sheet is to provide a containment for fluids absorbed by the product and to prevent the wetted product from leaking and staining the wearer's clothes. Therefore, the back sheet should be fluid-impermeable. However, the back sheet may allow passage of gases such as water vapor.

The back sheet can be made of a polyethylene (PE) film or a layer of dense nonwoven made of polypropylene (PP) fibres or a laminate of both. The back sheet may be made of a Mater-Bi film offered in the market by Polycart S.p.A. The film in 32 g/m² is suitable as a back sheet of absorbent pads. In a more preferred embodiment, the back sheet is made as a fibrous, hydrophobic wet-laid structure offered by Glatfelter company under the trade name Glatpure. The back sheet may comprise a film layer laminated with a nonwoven layer, forming a so-called textile back sheet.

Biodegradable back sheets are also possible. In such embodiments, the back sheet may be or may comprise a sheet of cellulose material or material of a cellulose derivative. The back sheet may be or may comprise a cellulose film such as a cellophane film. The back sheet may contain or may be coated with a softening agent such as glycerol.

In one embodiment, the back sheet comprises or consists of a cellulose film or a film of a cellulose derivative (the former being preferred), laminated with a nonwoven made of cellulose fibers, wherein said film laminated with a nonwoven preferably has a basis weight of 5 to 40 g/m², preferably 10 to 30 g/m², and even more preferably 15 to 25 g/m².

Alternatively, the back sheet may be a sheet made of or comprising cellulose fibrous material, said sheet having pores with a pore size not large enough for water droplet transmission, but large enough for water vapor transmission. Thus, the layer may be breathable. The pore size of said sheet may be smaller than 100 µm, preferably smaller than 50 µm. In one embodiment, the back sheet does not have relevant pores.

The absorbent product has, as explained above, a top sheet that is fluid permeable and may for that purpose, have pores. On the other hand, the back sheet is fluid impermeable. If the back sheet has pores, the median pores size of pores of the top sheet is at least 20 times, preferably at least 50 times, more preferably at least 100 times larger than the median pore side of pores in the back sheet. This embodiment covers the case wherein the back sheet does not have relevant pores.

To generate a suitable soft, pleasant feel of the back sheet, plasticizers can be added, such as glycerol. Antioxidants may also be included in the back sheet or film. Desired opacity of the back sheet can be achieved by addition of neutral, mineral fillers, such as talc, calcium carbonate or silica.

Such technology is described in "The Plasticization Effect of Glycerol and Water on the gelatinization of Wheat Starch by Gena Nashed et al. (April 2003, Starch - Stärke 55 (3-4), DOI: 10.1002/star.200390027). Another way to obtain such films is described in "Novel Starch/Chitosan/Aloe Vera Composites as Promising Biopackaging Materials" by Dagmara Baier et al., J. Polym. Environ 28, 1021-1039 (2020). Xu et al. describe in "Robust and Flexible Films from 100% Starch Cross-Linked by Biobased Disaccharide Derivative" the use of oxidized sucrose for crosslinking the starch (ACS Sustainable Chem. Eng. 2015, 3, 11, 2631-2639). Ning et al. describe the advantage of using citric acid in "Influence of Citric Acid on the Properties of Glycerol-Plasticized Cornstarch Extrusion Blends" (School of Science, Tianjin University, China, in Polymers & Polymer Composites, Vol. 15, No. 7, 2007).

A fibrous sheet as a back sheet can be produced by wet laid technology in which a suspension of fibers in water will be dewatered on a screen, whereby a fibrous web is formed and remains for further process. Papers, consisting of cellulose pulp fibers, are typically produced by such process. Wet-laid nonwovens can use other, longer fibers, e.g., fibers made of regenerated cellulose like viscose.

Depending on the manufacturing process such nonwovens can be wet-laid or airlaid made. A good description of wet-laid cellulosic nonwovens is given by Hemamalini in "Wet laying nonwovens using natural cellulosic fibers and their blends: process and technical applications. A review" (https://doi.org/10.1080/15440478.2019.1701606). Dry laid nonwovens made of cellulosic fibers are known to those skilled in the art. As an example, dry or air laid nonwovens made of cotton or viscose fibers by carding or Rando process are available in the market.

A viscose-based nonwoven with reduced binder content is described in EP 2138056 A3. A new approach to a viscose-based nonwoven is described in CN2012100077076A. This application covers a spun-laid viscose nonwoven which can be tight enough to provide a barrier function to fluids and a good permeability to water vapor.

A back sheet made of cellulosic fibers and best suitable for the use in this invention has a good breathability, will be non-permeable to fluids like urine and blood under in-use conditions and will preferably be hydrophobic on its inner (facing absorbent core) side.

The back sheet is preferably hydrophobic in nature, as the back sheet should provide a barrier to fluids and forms a container for them. Thus, the back sheet may be coated to provide a desired level of hydrophobicity. A person skilled in the art will appreciate several bio-materials which can be used as a treatment or coating to a wet-laid material and provide a hydrophobicity to it. A method that uses starch as a hydrophobicity (repellency) agent is described in WO2018050317.

Another requirement for a back sheet in absorbent hygienic products is softness which provides a pleasant feel for a use and lack of noise when a user moves. Both properties can be improved using a fiber mix containing at least several percent of short cellulosic fibers as well as a mechanical treatment to the sheet. Such treatment can comprise embossing, corrugating and similar deformations of plain web surface. As an example, a soft embossing of a fibrous structure is described in US 6,468,392. While this patent describes mostly hydrophilic fibrous structures, a combination with a hydrophobisation step will result in desired properties for a back sheet. Also, a use of so-called air-through dryer prior to the hydrophobisation step can improve the softness and "silence" of such web. A paper machine equipped with such air-through-dryer is described in US6398916. Also, a ring-rolling technology can be used as described in US 4,517,714.

The back sheet may have pores to make it breathable. The pore size should not be large enough for water droplet transmission, but large enough for water vapor transmission. The pore size of said sheet may be smaller than 100 µm, preferably smaller than 50 µm. In one embodiment, the back sheet does not have relevant pores.

Other possible materials for the back sheet are poly(butylene succinate co-butylene adipate) known as PBSA polymer which can be obtained from renewable feedstock such as glucose and sucrose. Also, thermoplastic starch (TPS) enhanced by additives and plasticizers can be used for producing a back sheet. A further example of a material usable for producing a back sheet is polyhydroxyalkanoates (PHA).

### Absorbent core

The absorbent core comprises at least the absorbent layer and the cover sheet covering the absorbent layer. The absorbent core serves to receive, store and bind body fluids. Typical absorbent layers comprise fibers and particles of superabsorbent polymers. While fibers provide fluid distribution and a certain absorbent core integrity, superabsorbent particles absorb and practically irreversibly store the fluid. Performance parameters of absorbent layers include speed of absorption, absorption capacity, and fluid retention.

The absorbent layer of the absorbent product of the invention generally comprises a superabsorbent polymer material, such as particles of partly neutralized and partly crosslinked polyacrylic acid. Another possible superabsorbent polymer material is or is based on carboxymethyl cellulose, such as the sodium salt of carboxymethyl cellulose. Also this superabsorbent polymer material is generally provided in the form of particles. The absorbent layer may comprise particles of superabsorbent polymer and at most 10 % by weight of fibers shorter than 10 mm, preferably shorter 8 mm. Alternatively or additionally, the absorbent layer comprises at least 40 % by weight, preferably at least 50 % by weight of said superabsorbent polymer or particles thereof.

The absorbent layer generally swells during uptake of water or body fluids. In the invention, the absorbent layer, when saturated with aqueous 0.9% NaCl solution, expands in z-direction (thickness) preferably at least by 3 times its thickness, preferably at least 4 times, e.g. within 5 minutes.

The absorbent layer may be an airlaid composite strip. Composite materials suitable for such an element are offered in the market e.g. by McAirlaid company under the trade name "SuperCore", e.g., as a T-301-S material consisting of 45% cellulosic pulp fibres and 55% of a superabsorbent polymer. The thickness of this material is, for example, 1.4 mm and its density 214 kG/qm. Another source of such airlaid composite materials is Domtar EAM located in Jesup, GA, USA.

The cover sheet is a generally lofty fibrous sheet that may, to some extent, contain superabsorbent polymer particles. The cover sheet is an extensible fibrous sheet. The cover sheet is preferably extensible, at least in cross direction, by at least 20%, preferably at least 30%, more preferably at least 40% in length without breaking. The property may be measured before including the cover sheet into the absorbent product using a tensile test machine as described in further detail below. The extensibility is preferably non-elastic or non-reversible to avoid or reduce, upon extending, a reversing force against the extension.

The cover sheet comprises fibers and preferably consists essentially of fibers. The cover sheet may consist of fibers and, optionally, superabsorbent polymer particles. The terms "consists of fibers" does not exclude the presence of dust and trash particles, such as dust and trash particles of cotton plants. The cover sheet may contain 20 % by weight or less of superabsorbent polymer particles, preferably from 1 to 15 % by weight, relative to the weight of the cover sheet, of superabsorbent polymer particles. In one embodiment, the cover sheet consists of fibers. In a preferred embodiment, the cover sheet consists of fibers and 20 % by weight or less (relative to the weight of the cover sheet) superabsorbent polymer particles. In a more preferred embodiment, the cover sheet consists of fibers and from 1 to 15 %, preferably from 2 to 10 %, by weight (relative to the weight of the cover sheet) superabsorbent polymer particles.

The fibers of the cover may have a mean length by number L(n) of at least 5 mm, preferably at least 8 mm, more preferably at least 10 mm, even more preferably at least 12 mm, and most preferably at least 15 mm.

In alternative embodiments, the fibers of the cover sheet comprise at least 50 % by weight fibers of a length of at least 7 mm, preferably at least 10 mm, more preferably at least 12 mm, even more preferably at least 14 mm, and most preferably at least 17 mm. Preferably, the fibers of the cover sheet comprise at least 70 %, more preferably at least 80% by weight fibers of a length of at least 7 mm, preferably at least 10 mm, more preferably at least 12 mm, even more preferably at least 14 mm, and most preferably at least 17 mm.

Fibers of the cover sheet are interconnected, preferably exclusively, by mechanical forces selected from fiber-to-fiber friction, entangling, and/or interlacing. As a result, under tension applied to the cover sheet, the fibers (including those interconnected by said mechanical forces) can move relatively to each other to some extent without breakage of said cover sheet. Consequently, the cover sheet is extensible and can accommodate a swelling of the absorbent layer due to uptake of body fluid. The extensibility is as described above. The cover sheet may be a sheet of fluffy wadding of said fibers.

The cover sheet can be made of any staple fiber, wherein said fibers may be fibers of PE, PP, polyethylene terephthalate (PET), cellulosic fibers, or mixtures thereof, preferably said fibers are cellulosic fibers. The cellulosic fibers may be cotton fibers, pulp fibers, and/or viscose fibers, preferably cotton fibers. The fibers are preferably laid down by usual textile techniques.

Cotton fibers having a suitable length for use in the invention are typically combers. They are available in the market e.g., from Barnhardt Manufacturing Company as Purified Organic Cotton Combers under the product code BKC10042BL1. The fiber length of this product is between 0.54 and 0.74 inches and the Micronaire is between 3.0 and 3.8 inches.

Similar lofty sheets made of cotton fibers and comprising superabsorbent polymer particles are described in US9398983 issued to Corman. Such sheet is usable in an absorbent core with good absorption by superabsorbent particles and fluid wicking by long cotton fibers. Also, the technology and machinery suitable to produce such layer is disclosed in that patent.

The cover sheet may comprise superabsorbent polymer. The superabsorbent polymer may be the same as indicated above. The cover sheet may contain 20 % by weight or less of superabsorbent polymer, preferably from 1 to 15 % by weight of superabsorbent polymer.

The cover sheet may have a basis weight of 20 to 200 g/m², preferably from 30 to 120 g/m², more preferably from 40 to 100 g/m².

In one embodiment, the cover sheet consists of (preferably bleached) cotton fibers mixed with particles of superabsorbent polymer in the amount indicated above and preferable having the above-mentioned characteristics of fiber length and extensibility.

### Absorbent product

A plan view (viewed along the z-axis) on the inner side of the absorbent product is shown in Fig. 1 and a cross-section thereof along line A-A is shown in Fig. 2. The shape of the absorbent product in the plan view of Fig. 1 is longitudinal and may be somewhat narrower in the middle to allow a good body fit e.g. for placement in a panty.

The absorbent layer 27 is generally placed and bonded to the back sheet 29, generally in the middle in y-direction of the back sheet. The position of the absorbent layer in the plan view of Fig. 1 is indicated by a dashed line. The shape of the absorbent layer in the view of Fig. 1 may be rectangular or may be somewhat narrower in the middle and curved at the short ends thereof. The absorbent layer should have a maximum absorbing ability at locations that face body parts from where discharge of body fluids (notably urine or blood) are to be expected.

The cover sheet 25 covers the absorbent layer and extends beyond the absorbent layer on at least two opposing sides of the absorbent layer. The at least two opposing sides are preferably the lateral sides, i.e. the left- and right-hand sides in Figs. 2. The cover sheet may also extend beyond the absorbent layer on the other (short) sides of the absorbent layer. The cover sheet preferably has a width in cross (y-) direction of at least 4 mm, preferably at least 12 mm, larger than the width (in cross-direction) of the absorbent layer, at least in a central part of the product with respect to the x-direction. Preferably, the cover sheet has a width in cross (y-) direction of at least 10, more preferably at least 12 mm, larger than the width (in cross-direction) along the entire length of the absorbent layer in x-direction. "Along the entire length of the absorbent layer" means that the condition is fulfilled at each position along the x-dimension and measured along the y-direction at each position of the x-dimension of the absorbent layer. The extension of the cover sheet is measured in a planar shape of the absorbent product in top view, i.e. viewed along the z-axis and in a projection on the x-y plane, as shown in the cross-section of Fig. 3. Generally, the extending parts of the cover sheet are bent down to be bonded to the back sheet. The extending part of the cover sheet is indicated in Fig. 3 by distance e. Since the absorbent product is generally symmetrical with respect to a mirrow plane defined by the x- and z axis, the length of e is generally half of the width by which the width of the cover sheet extends beyond the width of the absorbent layer in y-direction.

Preferably, the portions of the cover sheet extending beyond the absorbent layer are bonded to the back sheet as indicated in Figs. 2 and 3, forming bonding parts 31 (on both sides). Bonding the extending parts of the cover sheet to the back sheet provides stability and integrity to the absorbent product in the state before use as well as in use after body fluid has been absorbed by the absorbent layer.

The absorbent layer swells upon absorption of body fluid and may increase its thickness several-fold. It is a key feature of the present invention that the cover sheet allows maintaining stability and integrity of the product when in use, evening after substantial swelling of the absorbent layer. This effect is achieved by the structure and extensibility of the cover sheet as described above.

The top sheet 23 covers both the cover sheet and the absorbent layer. It generally has similar extensions and shape as the back sheet. The top sheet is bonded to the back sheet along the periphery of the product, forming seal area 13 indicated in Figs. 1, 2 and 3. Bonding may be achieved by any suitable glue and/or by embossing. Seal area 13 generally has high flexibility, since it comprises less sheets than central portions of the product. The seal area encloses the absorbent core. The outer solid line 1 in Fig. 1 is the peripheral edge of the absorbent product. The inner solid line 3 in Fig. 1 is the seal line that is the inner boundary of the seal area 13. The width of seal area 13 may be or may vary between 3 and 25 mm, measured orthogonal to the peripheral edge of the absorbent product. Its width may vary along the periphery of the product and may, for example, be larger in the middle area of the product, e.g. near line A-A shown in Fig. 1 at the lateral sides (left and right sides in Fig. 1). The bonding of the top sheet and back sheet in the seal area 13 may be achieved using a glue or bonding agent. Alternatively, the bonding may be achieved by embossing.

On opposite longitudinal (lateral) sides, seal area 13 may comprise lateral portions that contain elastic elements 35 arranged in longitudinal direction in the lateral portions. When the absorbent product accommodates a flat planar shape, the elastic elements 35 are in an extended condition and exert a contracting force in the lateral portions to contract them and to move them upwards (in z-direction). Thereby, the absorbent product approaches a cup shape at least in the middle part (of the x-direction), such that the lateral portions move upward in z-direction. The cup-shape improves tight fit of the absorbent product to the body of the wearer to provide containment for exuded body fluid.

The elastic elements 35 may be elastic yarns, e.g. Lycra yarns. The absorbent product may have one elastic element in each lateral side. Preferably, the absorbent product may have two or more elastic elements 35 in each lateral side. When the absorbent product adopts a flat planar shape, the elastic elements are extended to at least 120%, preferably at least 150%, in length in x-direction relative to a relaxed state of the elastic elements. The elastic elements may be inserted in the lateral sides of the seal area, upon production of the absorbent product, in between the top and back sheets before bonding the top and back sheets together to form the seal area. Thereby, the elastic elements can be enclosed in the seal area in extended condition. The lateral parts 15 are those parts of the seal area where the elastic elements are in extended conditions by way of being bonding between the top and back sheets.

In order to exert an efficient cup-forming effect, the elastic elements 35 should have a certain minimum distance, in y-direction, from the absorbent layer at least in a central portion, with respect to the x-direction, of the absorbent product, preferably along the entire lateral sides of the product. The "central portion" means a portion of a length of at least 5 cm, preferably at least 8 cm, and most preferably at least 10 cm in length in x-direction, centered in the middle of the absorbent product in x-direction. In top view of the absorbent product or in the y-z-plane, the elastic element 35 has (on each lateral side of the product) an edge facing, in y-direction, the absorbent layer, and the absorbent layer has an edge facing, in y-direction, the elastic element. The minimum distance d indicated in Fig. 3 is, when the absorbent product adopts a flat planar shape, at least 5 mm, preferably at least 7 mm, and more preferably at least 10 mm from the edge of the (innermost) elastic element to the (edge of the) absorbent layer. If the absorbent product has more than one elastic element on each lateral side, it is understood that the distance is measured from the innermost (closest) elastic element to the absorbent layer, as shown in Fig. 3 that also shows how distance d is measured from edge-to-edge from the innermost elastic element to the absorbent layer.

The distance, in cross direction, from the edge of the absorbent layer 27 to the seal line 3, i.e. the closest point of seal area 13, may be, in cross direction, at least 4 mm, preferably at least 6 mm, more preferably at least 8 mm, when the absorbent product adopts a flat, planar shape. Said minimum distance is present preferably at least on both lateral sides at least in said central portion of the absorbent product mentioned above.

The absorbent product may further comprise an acquisition and distribution layer (ADL) between the top sheet and the cover sheet. ADLs in absorbent products were introduced to provide an intermediate storage for body exudate, providing more time for absorbent cores (mostly those containing high concentrations of superabsorbents) to slowly absorb the exudate. ADLs of prior art products are typically made of thick, bulky, and resilient nonwovens. In many products, the nonwovens are made of polyester fibers, as they offer better stiffness and regain their original volume when pressure applied to them is released. ADLs generally allow for spreading the fluid within the plane of the layer by the wicking ability of the ADL, as well as in the thickness (z) direction of the ADL. The thickness and resilience are important for ADLs. Even if thin, well compressed fibrous layers can provide a good wicking and distributing performance, the intermediate storage of fluid before its absorbtion by superabsorbent polymer may need a certain volume and thus the thickness of ADL.

The optional ADL of the absorbent product may be or may consist of one layer providing the acquisition and distribution function, or may comprise two or more layers, one providing mainly the acquisition and another one providing mainly the distribution function. Preferably, the ADL is a sheet in the sense of the invention, i.e. the ADL is a sheet made of polysaccharide. The ADL may consist of polysaccharide, preferably cellulose or a cellulose derivative (such as cross-linked cellulose). The ADL may be or may comprise a fibrous batt. A batt is a fibrous structure that is a loose assemblage of fibers. In one embodiment, the ADL is or comprises at least one sheet made of cellulose material, preferably made of cellulose fibers, said fabric preferably having a density of from 0.10 to 0.40 g/cm³, preferably of from 0.20 g/cm³ to 0.35 g/cm³ and being a sheet of (bathroom) tissue paper.

The absorbent product of the invention may contain a pH regulating agent to avoid decomposition of urine to ammonia. The pH regulating agent may be a solid organic acid or an acidic salt of an organic acid. Examples of organic acids are citric acid, tartaric acid, and ascorbic acid. Examples of acidic salts of organic acids are sodium dihydrogen citrate and mono-sodium tartrate. A preferred pH regulating agent is sodium dihydrogen citrate. The pH regulating agent may be included into or on the back side of the top layer. If the absorbent product contains a ADL, the pH regulating agent may be inserted into the ADL. The pH regulating agent may be present in an amount of at least 0.1 g per product, preferably from 0.2 to 1 g per product.

The absorbent product of the invention may be an absorbent hygiene product such as a sanitary napkin (also referred to as menstrual pad), panty liner, or incontinence pad. The absorbent product of the invention can - for the discretion and the comfort of the wearer - be packaged individually. Accordingly, the invention provides a packaged absorbent product comprising the absorbent product wrapped in a film made of, e.g. cellulose material or a material of a cellulose derivative, such as cellophane, or wrapped in a sheet made of cellulose fibers.

### Test methods

### Thickness of the absorbent layer

The thickness of absorbent layer in both dry and saturated wet condition can be measured with a micrometer provided the pressure of the measuring foot. We used Luhne Digital Micrometer Model 49-56 which allows thickness measurement between 0 and 10 mm.

### Extensibility of the cover sheet

This test can be performed on practically every tensile test machine. The inventors used a Chatillon Model LTCM 100 + E - DFE tester. The settings were: Clamp width: 55 mm, Distance of clamps in starting position: 14 mm. Speed of the clamps at testing: 500 mm per minute. Position of sample central point (Middle of the length and middle of the width) in the middle of clamps and 7 mm distance from each clamp. For the invention, the measurement in cross direction of the cover sheet is more relevant.

### Density

Density is the mass per unit volume of a material. Density and specific gravity of the material can be measured according to ASTM D792 and ISO 1183 standardized test methods.

### Basic weight

Basic weight is the weight of a surface unit of a product. It can be measured based on ASTM D646-96, reapproved 2001.

### Fiber length

Mean fiber length by number L(n) and by weight L(w) is measured by the Uster^{®} *Afis Pro 2* instrument according to the manufacturer's instructions. Repeated measurements of the same sample can be used to increase the statistical accuracy of the measurement. Measurements are conducted at 20 ± 2°C and 65 ± 2 % relative humidity. Short fiber content is determined at shorter than 12.7 mm. The instrument is primarily used for cotton fibers, but can be used for other fibers as well.

### Reference Example

A product according to this invention was tested in the lab. The absorbent layer made of an airlaid composite and having a thickness of 1.2 mm was saturated with 0.9% aqueous NaCl solution. After 15 minutes, the thickness of 7.1 mm was measured.

The lofty fibrous layer as described before was taken from the manufacturing process before its entry in to the "marriage" with other product elements and placed in the tensile test machine. The elongation at break measured was in average 48.75 mm which corresponds to 348%. The force at break was as low as 0.9 N. As a comparison, the same measurement was done for the absorbent layer: The elongation at break was 10,0 mm which corresponds to 71% and the force at break was 14.2 N.

## Claims

1. An absorbent product comprising a fluid-permeable top sheet, a fluid-impermeable back sheet, and an absorbent core positioned between said top sheet and said back sheet in a central portion of the absorbent product,
said absorbent core comprising an absorbent layer and a cover sheet covering the absorbent layer, said cover sheet extending beyond the absorbent layer on at least two opposing sides of the absorbent layer,
**characterized in that** said cover sheet is an extensible fibrous sheet.

2. The absorbent product according to claim 1, wherein the fibers of the cover sheet have a mean length by number L(n) of at least 5 mm, preferably at least 8 mm, more preferably at least 10 mm, even more preferably at least 12 mm, and most preferably at least 15 mm.

3. The absorbent product according to claim 1 or 2, wherein the absorbent product has an elongated and essentially flat shape or is capable of accommodating an elongated flat shape, wherein the cover sheet extends beyond the absorbent layer at least along the lateral sides of the absorbent layer, the lateral sides extending essentially parallel to the elongation direction.

4. The absorbent product according to any one of claims 1 to 3, having a longitudinal x-direction or axis extending along the elongation direction of the elongated absorbent product, a cross (or transverse) y-direction or axis within the flat plane orthogonal to longitudinal x-direction, and a thickness z-direction or axis orthogonal to both the longitudinal and transverse directions,
said cover sheet having a width in cross direction at least 4 mm, preferably at least 12 mm, larger than the width of the absorbent layer as viewed along the z-direction on the product in planar shape, at least at a central part along the x-direction of the product, preferably along the entire length in x-direction of the absorbent layer.

5. The absorbent product according to any one of claims 1 to 4, wherein the cover sheet is affixed to the back sheet at its portions extending beyond the absorbent layer so as to enclose the absorbent layer between the cover sheet and the back sheet.

6. The absorbent product according to any one of claims 1 to 5, wherein said cover sheet, before attaching or integrating it into said absorbent product, is extensible, at least in cross direction, by at least 20%, preferably at least 30%, more preferably at least 40% in length without breaking.

7. The absorbent product according to any one of claims 1 to 6, wherein the cover sheet is a sheet of fluffy wadding of said fibers, and/or said fibers are interconnected exclusively by mechanical forces selected from fiber-to-fiber friction, entangling, and interlacing, wherein under tension applied to the cover sheet, the fibers can move relatively to each other without breakage of said cover sheet.

8. The absorbent product according to any one of claims 1 to 6, wherein said fibers are fibers of polyethylene (PE), polypropylene (PP), polyethylene terephthalate (PET), cellulosic, or mixtures thereof, preferably said fibers are cellulosic fibers, wherein said cellulosic fibers are cotton fibers, pulp fibers, and/or viscose fibers, preferably cotton fibers.

9. The absorbent product according to any one of claims 1 to 7, wherein said cover sheet contains 20 % by weight or less of superabsorbent polymer particles, preferably from 1 to 15 % by weight of superabsorbent polymer.

10. The absorbent product according to any one of claims 1 to 8, wherein said cover sheet has a basis weight of 20 to 200 g/m², preferably from 30 to 120 g/m².

11. The absorbent product according to any one of claims 1 to 11, wherein said absorbent layer comprising particles of superabsorbent polymer and at most 10 % by weight of fibers shorter than 10 mm, preferably shorter 8 mm.

12. The absorbent product according to any one of claims 1 to 11, wherein said top sheet and said back sheet are bonded to each other along the periphery of the absorbent product to form a seal area 13.

13. The absorbent product according to a claim 12, wherein said seal area 13 comprises lateral portions extending on and along lateral sides of said absorbent product, said lateral portions containing elastic elements arranged in x-direction and in extended condition when the absorbent product accommodates a flat planar shape, said elastic elements exerting a contracting force on the lateral portions to contract them and to form the absorbent product to a cup-shape when said elastic elements are relaxed.

14. The absorbent product according to claim 13, wherein, when in a flat shape/state of the absorbent product, the elastic elements are extended to at least 120%, preferably at least 150%, in length compared to a relaxed state of the elastic elements.

15. The absorbent product according to claim 13 or 14, wherein the elastic element closest to the absorbent layer in y-direction of the product in planar shape is distanced from the absorbent layer in cross direction by at least 5 mm, preferably by at least 7 mm, when the absorbent product is formed into a flat shape.
